# EUROPÄISCHE PATENTANMELDUNG

(11) **EP 2 402 747 A2**
(43) Veröffentlichungstag der Anmeldung: **04.01.2012**
(21) Anmeldenummer: 11005175.2
(22) Anmeldetag: 25.06.2011
(51) Int. Cl.: G01N 33/00

(54) **Analyseschrank sowie Verfahren zur Analyse eines Messgases aus einem grosstechnischen Prozess**

(30) Priorität: 02.07.2010 DE 102010025926
(71) Anmelder: Johnson Matthey Catalysts (Germany) GmbH, 96257 Redwitz (DE)
(72) Erfinder: Ziesmann, Martin, 96257 Redwitz (DE); Strobl, Klaus, 86570 Inchenhofen (DE); Kügel, Daniel, 96264 Altenkunstadt (DE)
(74) Vertreter: FDST Patentanwälte

(57) **Zusammenfassung**

Der Analyseschrank (2) dient zur Analyse insbesondere eines aus einem großtechnischen Prozess stammenden Messgases (G) und umfasst eine Gasaufbereitungsvorrichtung (12) sowie eine Analyseeinrichtung (14) für das Messgas (G). DerAnalyseschrank (2) ist in mehrere Schrankteile (4,6,8,10) untergliedert, wobei die elektrischen Komponenten in einem Elektro-Schrankteil (4) angeordnet sind und dieser zu den weiteren Schrankteilen (6,8,10) sowie zur Umgebung hin abgeschottet ist. Hierdurch ist insgesamt eine kompakte integrale Baueinheit geschaffen unter gleichzeitiger Gewährleistung der Einhaltung insbesondere der EG-Maschinenrichtlinie.

## Beschreibung

Die Erfindung betrifft einen Analyseschrank sowie ein Verfahren zur Analyse eines insbesondere aus einem großtechnischen Prozess stammenden Messgases, insbesondere ein Abgas aus einem Verbrennungsprozess.

Bei großtechnischen Prozessen, insbesondere Verbrennungsprozessen wird zur Steuerung und Regelung der Verbrennung regelmäßig die Zusammensetzung der Verbrennungsabgase im Rahmen einer so genannten "Rauchgasanalytik" analysiert. Unter einer großtechnischen Anlage werden insbesondere Verbrennungsanlagen mit einer thermischen Leistung von mindestens 50 kW und vorzugsweise von mindestens 100 kW bis hin in den MW- oder GW-Bereich verstanden. Derartige Verbrennungsanlagen sind beispielsweise Verbrennungsmotoren auch in Anlagen zur Energieerzeugung oder im Bereich der Marine. Daneben werden hierunter auch beispielsweise Müllverbrennungsanlagen und allgemeine Strom erzeugende Anlagen verstanden.

Zur Analyse der Abgase wird üblicherweise ein Teilstrom des Abgases während des Betriebs der Anlage abgezweigt und als Messgas einer Analyseeinrichtung zugeführt. Diese umfasst beispielsweise unterschiedliche Analysegeräte, die in den Messschränken zusammen mit entsprechenden Auswerteeinheiten angeordnet sein können. Derartige Messschränke genügen jedoch oftmals nicht den Anforderungen und vorgegebenen Spezifikationen oder Normen, beispielsweise der sogenannten EG-Maschinenrichtlinie 2006/42/EG sowie 98/37/EG.

Der Erfindung liegt die Aufgabe zugrunde, eine zuverlässige und sichere Analyse derartiger Messgase entsprechend vorgegebener Spezifikationen zu ermöglichen.

Die Aufgabe wird gemäß der Erfindung gelöst durch einen Analyseschrank, der zur Analyse eines Messgases dient, welches insbesondere aus einem oben beschriebenen großtechnischen Prozess stammt. Der Analyseschrank ist in mehrere Schrankteile aufgeteilt, die gemeinsam den Analyseschrank bilden. Der Analyseschrank weist also ein gemeinsames Traggerüst mit einem Außengehäuse auf, innerhalb dessen die verschiedenen Schrankteile angeordnet sind. Die verschiedenen Schrankteile sind ein Elektro-Schrankteil mit einer elektrischen Schaltkastenanordnung, ein Gas-Schrankteil mit einer Gasaufbereitungsvorrichtung sowie ein Analyse-Schrankteil mit einer Analyseeinrichtung zur Messgasanalyse. Von besonderer Bedeutung ist nunmehr, dass der Elektro- und vorzugsweise ebenfalls der Analyse-Schrankteil zum Gas-Schrankteil hin und vorzugsweise insgesamt zur Umgebung hin abgeschottet sind. Unter abgeschottet wird hierbei verstanden, dass innerhalb des Elektro- und Analyse-Schrankteils jeweils ein von dem Gas-Schrankteil und vorzugsweise auch von der Umgebung abgeschottetes Innenraumklima eingestellt ist mit insbesondere vorgegebener Temperatur, vorgegebener Feuchtigkeit und/oder vorgegebener Gaszusammensetzung. Unter abgeschottet ist daher insbesondere auch zu verstehen, dass kein (nennenswerter) Gasaustausch zwischen den Schrankteilen erfolgt. Die einzelnen Schrankteile sind daher zueinander abgedichtet. Es handelt sich um abgeschlossene Teilräume innerhalb des Analyseschrankes.

In dem Elektro-Schrankteil sind vorzugsweise sämtliche elektrischen oder elektronischen Geräte, insbesondere für die Einspeisung, Verteilung und Steuerung von Strom untergebracht. Auch ist im Elektro-Schrankteil ein zentraler Steuerteil für die Kommunikation und den Datenaustausch zwischen allen Komponenten des Analyseschranks enthalten. All diese einzelnen Vorrichtungen sind daher im Elektro-Schrankteil angeordnet und bilden eine Anordnung, die vorliegend als eine elektrische Schaltkastenanordnung bezeichnet wird. Sämtliche außerhalb des Elektro-Schrankteils angeordneten weiteren elektrischen oder elektronischen Bauteile, wie beispielsweise die in der Analyseeinrichtung zwingend vorhandenen Messsensoren, sind zumindest für den Einsatz in einer belasteten Umgebung ausgelegt, beispielsweise indem die Geräte, insbesondere die Abdichtung der Gehäuse oder der elektrischen Zuleitungen dieser Geräte, den entsprechenden Schutzarten gemäß der IP-Klassifizierung entsprechen, beispielsweise der IP54-Klassifizierung.

Der besondere Vorteil des abgeschotteten Elektro-Schrankteils ist darin zu sehen, dass durch diese Maßnahme, also durch die Trennung von der Umgebung, zuverlässig vermieden ist, dass die elektrischen Bauteile der Schaltkastenanordnung mit schädlichen gasförmigen oder flüssigen Stoffen in Kontakt kommen. Dadurch ist die Zuverlässigkeit sowie die Lebensdauer verbessert und insbesondere die Einhaltung von Normen, insbesondere der EG-Maschinenrichtlinie gewährleistet, bei gleichzeitiger Integration aller Analysekomponenten innerhalb eines gemeinsamen Schrankes.

Durch die in bevorzugter Ausgestaltung ergänzend vorgesehene Abschottung auch des Analyse-Schrankteils wird zudem der besondere Vorteil erzielt, dass die Analyse-Geräte innerhalb eines definierten Klimas angeordnet sind und somit möglichst unverfälschte Messergebnisse abgeben.

Schließlich ist durch die zusätzlich im Analyse-Schrank vorgesehene Gasaufbereitungsvorrichtung gewährleistet, dass der Analyseeinrichtung Messgase in definiertem Zustand (beispielsweise definierte Feuchtigkeit) zugeführt werden, was ebenfalls für unverfälschte Messergebnisse von Vorteil ist.

Gemäß einer bevorzugten Ausgestaltung ist hierzu vorgesehen, dass zwischen dem Elektro- und dem Gas-Schrankteil eine dichte Trennwand mit einer abgedichteten Elektrodurchführung zur Durchführung zumindest einer elektrischen Leitung (Elektrokabel) vorgesehen ist. Steuer-, Daten-, und/oder elektrische Versorgungsleitungen, die in oder durch den Gas-Schrankteil hindurchgeführt werden, werden daher über die abgedichtete Elektrodurchführung geführt, d.h. ein Durchbruch für die Durchführung des Elektrokabels ist in geeigneter Weise beispielsweise mit Hilfe eines Abdichtelements abgedichtet. Hierdurch wird die Aufrechterhaltung des abgeschotteten Teilraums gewährleistet.

Entsprechend ist in zweckdienlicher Ausgestaltung auch zwischen dem Analyseund dem Gas-Schrankteil eine dichte Trennwand mit einer abgedichteten Gasdurchführung für das aufbereitete Messgas vorgesehen. Typischerweise wird hierzu eine Messgasleitung beispielsweise über einen Flansch durch die Trennwand hindurchgeführt.

Zweckdienlicherweise ist im Analyse-Schrankteil eine zweite abgedichtete Gasdurchführung für das Messgas vorgesehen, über die das Messgas nach Durchlaufen der Analyseeinrichtung wieder aus dem Analyse-Schrankteil herausgeführt wird. Vorzugsweise dient diese zweite Gasdurchführung zur Rückführung des Messgases in den Gas-Schrankteil und wird dort durch oder über Komponenten der Gasaufbereitungsanlage geführt, bevor das Messgas beispielsweise an die Umgebung abgegeben wird.

Innerhalb der Gasaufbereitungsvorrichtung sind üblicherweise mehrere Komponenten zur Gaswäsche- und/oder Gasaufbereitung vorgesehen. Diese sind je nach Bedarf und insbesondere in Kombination ein Gaswäscher zur Reinigung des Messgases von Verunreinigungen, ein Filter, insbesondere Staub- oder Partikelfilter zur Reinigung des Gases von in ihm enthaltenen Feststoffpartikeln, eine Einrichtung zur Be- oder Entfeuchtung des Messgases, um eine definierte Feuchtigkeit einzustellen, ein Kühler für das Messgas, um dieses auf eine definierte Temperatur zu temperieren sowie weiterhin Ventile und Pumpen zur Steuerung des Gasflusses. Weiterhin ist üblicherweise ein Kondensatbehälter zum Sammeln der im Messgas enthaltenen auskondensierten Feuchtigkeit vorgesehen.

Gemäß einer bevorzugten Weiterbildung ist ein zusätzlicher Kühl-Schrankteil vorgesehen, in dem zumindest Teile einer Wärmerückkühlanlage zur Temperierung des Analyseschranks auf eine vorgegebene Betriebstemperatur vorgesehen ist. Die Wärmerückkühlanlage kann allgemein als eine Anlage zur Klimatisierung der jeweiligen Teilräume des Analyseschranks angesehen werden, mit deren Hilfe also beispielsweise sowohl die Temperatur als auch die Feuchtigkeit in den einzelnen Schrankteilen auch gesondert eingestellt wird. Durch die Integration auch dieser Wärmerückkühlanlage ist insgesamt eine abgeschlossene, kompakte Schrankeinheit ausgebildet, die vollständig betriebsfähig ist, ohne dass ein großer zusätzlicher Installationsaufwand erforderlich wäre. Die Montage und Installation eines derartigen Analyseschranks ist daher vergleichsweise einfach.

In bevorzugter Ausgestaltung ist jedem der einzelnen Schrankteile ein eigenes Traggestell oder Traggerüst zugewiesen, wobei einzelne Tragelemente, wie beispielsweise Tragprofile, von benachbarten Schrankteilen gemeinsam benutzt werden können. Vorzugsweise sind zwei Schrankteile, insbesondere der Gassowie der Analyse-Schrankteil, übereinander angeordnet, während der dritte Schrankteil, beispielsweise der Elektro-Schrankteil, neben diesen beiden übereinander gestapelten Schrankteilen angeordnet ist. Bei ergänzender Anordnung des Kühl-Schrankteils ist dieser in vorzugsweise zwischen den anderen Schrankteilen angeordnet. Der Elektro-Schrankteil ist dadurch noch einmal zusätzlich vom Gas-Schrankteil getrennt. Ergänzend ist hierdurch die Temperierung oder Klimatisierung der einzelnen Teilräume der Schrankteile vereinfacht. In bevorzugter Ausgestaltung ist nämlich vorgesehen, dass die Wärmerückkühlanlage für jeden der einzelnen Schrankteile einen Wärmetauscher aufweist, der vorzugsweise jeweils an einer Trennwand des Kühl-Schrankteils zum jeweils benachbarten Schrankteil anliegt. Alternativ besteht auch die Möglichkeit, dass wiederum abgedichtete Durchführungen beispielsweise zu einem in den weiteren Schrankteilen angeordneten Wärmetauschern vorgesehen sein können.

Im Hinblick auf den konstruktiven Aufbau ist in zweckdienlicher Ausgestaltung weiterhin vorgesehen, dass die einzelnen Schrankteile jeweils über Türen zugänglich sind, um im Falle von Wartungsarbeiten einen einfachen Zugang zu ermöglichen. Zweckdienlicherweise ist die jeweilige Tür jeweils frontseitig angeordnet und überdeckt insbesondere zumindest nahezu die gesamte Frontseite des jeweiligen Schrankteils.

Die Aufgabe wird gemäß der Erfindung weiterhin gelöst durch ein Verfahren zur Analyse eines Messgases gemäß Anspruch 11. Die im Hinblick auf den Analyseschrank angeführten Vorteile und bevorzugten Ausgestaltungen sind sinngemäß auch auf das Verfahren zu übertragen.

Ein Ausführungsbeispiel der Erfindung wird im Folgenden anhand der Figuren näher erläutert. Es zeigen jeweils in vereinfachten Darstellungen:
- Fig. 1: eine Frontansicht auf einen Analyseschrank, und
- Fig. 2: ein stark vereinfachtes Blockschaltbild des Analyseschranks mit den darin angeordneten Komponenten.

Der in den Figuren dargestellte Analyseschrank 2 dient allgemein zur Analyse eines Messgases G. Das Messgas G stammt aus einer hier nicht näher dargestellten insbesondere großtechnischen Anlage, insbesondere eine Verbrennungsanlage. Das bei der Verbrennung entstehende Rauchgas wird üblicherweise zur Kontrolle, Steuerung und Regelung des Verbrennungsprozesses im Hinblick auf seine Zusammensetzung analysiert. Hierzu wird ein Teil des Rauchgases abgezweigt und dem Analyseschrank 2 als Messgas G zugeführt.

Die Verbrennungsanlagen können sowohl Motoren, wie beispielsweise Gasmotoren, Dieselmotoren, etc. aber auch herkömmliche Verbrennungsstätten, wie beispielsweise Müllverbrennungsanlagen sein. Über den Analyseschrank 2 wird eine komplette integrierte Einheit bereitgestellt, durch die die Analyse des Messgases G zuverlässig und in Übereinstimmung mit den geforderten Bestimmungen Richtlinien und Normen durchgeführt werden kann.

Der in den Figuren dargestellte Analyseschrank 2 weist mehrere nebeneinander bzw. übereinander angeordnete Schrankteile auf, nämlich einen Elektro-Schrankteil 4, einen Gas-Schrankteil 6, sowie einen Analyse-Schrankteil 8. Dieser ist im Ausführungsbeispiel unterhalb des Gas-Schrankteils 6 angeordnet, die gemeinsam eine Art Turm bilden. Im Ausführungsbeispiel ist ergänzend zwischen dem Elektro-Schrankteil 4 und den aufeinander getürmten Schrankteilen 6,8 noch ein Kühl-Schrankteil 10 angeordnet.

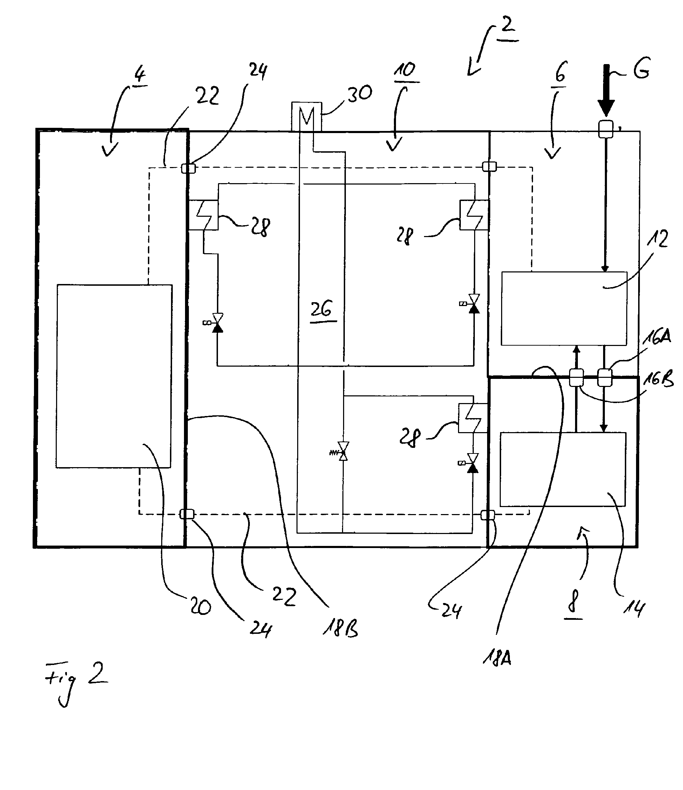

Dem Gas-Schrankteil 6 wird das Messgas G zugeführt. Innerhalb dieses Gas-Schrankteils ist eine in der Fig. 2 nur schematisch dargestellte Aufbereitungsvorrichtung 12 angeordnet. In dieser wird das Messgas G aufbereitet und einer definierten Reinheitsstufe zugeführt, bevor das Messgas G anschließend zur Analyse einer im Analyse-Schrankteil 8 angeordneten Analyseeinrichtung 14 zugeführt wird. In der Gasaufbereitungsvorrichtung 12 wird daher das Messgas G gereinigt, insbesondere von Stäuben und Partikeln, aber auch von in ihm enthaltenem Kondensat befreit. Ergänzend wird das Messgas G entwässert, also auf einen gewünschten Feuchtigkeitsgehalt getrocknet. Hierzu enthält die Gasaufbereitungsvorrichtung 12 in hier nicht näher dargestellter Weise Ventile, Pumpen zur Steuerung des Messgasstroms G, unter Druck stehende Befeuchtergefäße, Gaswäscher, wie beispielsweise Waschflaschen für das Gas, Filter, insbesondere Staub- oder Partikelfilter, Kühlaggregate zur Kühlung des Gases und zum Entwässern sowie Behälter für anfallendes Kondensat.

Im Gas-Schrankteil können - da dieser nicht weiter zur Umgebung hin abgeschottet ist - gasförmige sowie auch flüssige Medien in der Raumluft in variierenden, nicht bekannten Konzentrationen enthalten oder eingetragen sein. Die einzelnen Komponenten der Gasaufbereitungsvorrichtung 12, wie beispielsweise die Kühlaggregate, die Pumpen etc. sind daher entsprechend von geforderten Schutzklassifikationen, beispielsweise entsprechend der Klassifikation IP54 oder IP55 etc. ausgelegt. Der Gas-Schrankteil 6 mit der Gasaufbereitungsvorrichtung 12 ist dadurch insgesamt insbesondere derart konzipiert, dass die gemäß der EG-Maschinenrichtlinie 2006/42/EG sowie 98/37/EG geforderten Schutzklasse für diesen Teilbereich erfüllt sind.

Das aufbereitete Messgas G wird über eine abgedichtete erste Gasdurchführung 16A der Analyseeinrichtung 14 zugeleitet. Hierzu ist eine entsprechende Rohrleitung vorgesehen, die über die Gasdurchführung 16A durch eine Trennwand 18A durchgeführt ist. Nach der Analyse des Messgases G wird dieses wieder durch die Trennwand 18A und über eine zweite Gasdurchführung 16B zurück in den Gas-Schrankteil 6 geleitet.

Die Analyseeinrichtung 14 enthält hier nicht näher dargestellte Analysegeräte. Diese Geräte sind üblicherweise sehr empfindlich und besitzen oftmals auch eine geringe Schutzklasse, sind daher beispielsweise nicht zum Einsatz in einer feuchten Umgebung geeignet. Zudem erfordern sie üblicherweise auch ein aufbereitetes Messgas G. Aufgrund einer Abschottung des Analyse-Schrankteils 8 sowie der zuvor erfolgten Gasaufbereitung sind die üblichen Analysegeräte innerhalb des Analyse-Schrankteils 8 problemlos unter Einhaltung der entsprechenden Schutzklassen einsetzbar.

Im Elektro-Schrankteil 4 sind alle elektrischen und elektronischen Komponenten innerhalb einer so genannten Schaltkastenanordnung 20 zusammengefasst, die für die Einspeisung, Verteilung und Steuerung des für den Betrieb des Analyseschranks erforderlichen Stroms notwendig sind. Diese Komponenten sind insbesondere Sicherungen, Schutzschalter, etc. Ergänzend ist in dieser Schaltkastenanordnung auch eine zentrale Steuereinheit zur Steuerung der Komponenten des Analyseschranks sowie des gesamten Betriebs des Analyseschranks vorgesehen. Diese Steuereinheit kommuniziert insbesondere mit den weiteren elektrischen / elektronischen Komponenten des Analyseschranks 2 und ist mit diesen über entsprechende Daten- und Steuerleitungen verbunden. Außerhalb des Elektro-Schrankteils 4 befinden sich daher vorzugsweise nur solche elektrischen oder elektronischen Komponenten, die zwingend beispielsweise im Gas-Schrankteil 6, im Analyse-Schrankteil 8 oder im Kühl-Schrankteil 10 enthalten sein müssen. Dies sind beispielsweise Pumpen, elektrische Ventile etc. sowie elektrische Analysegeräte für die Analyse des Messgases G. Eventuelle Auswerteeinheiten zur Auswertung der Messergebnisse sind jedoch vorzugsweise nicht im Analyse-Schrankteil 8 sondern im Elektro-Schrankteil 4 angeordnet.

Von der Schaltkastenanordnung 20 gehen elektrische Leitungen 22, nämlich Versorgungs- Steuer- und/oder Datenleitungen ab und werden zu den entsprechenden Komponenten in den weiteren Schrankteilen 6,8,10 geführt. Beispielhaft sind zwei Leitungen oder Leitungspakete dargestellt, die einerseits zu der Analyseeinrichtung 14 und andererseits zu der Gasaufbereitungsvorrichtung 12 führen. Diese sind in Fig. 2 gestrichelt dargestellt. Diese Leitungen 22 werden durch eine dichte Trennwand 18B des Elektro-Schrankteils 4 über abgedichtete Elektrodurchführungen 24 aus dem Elektro-Schrankteil 4 herausgeführt. Über weitere Elektrodurchführungen 24 werden die Leitungen 22 in die weiteren Schrankteile wieder eingeführt. Insbesondere bei der Einführung in den Analyseschrankteil 8 ist ebenfalls wieder eine dichte Elektrodurchführung 24 durch eine abgedichtete Trennwand vorgesehen.

Im Ausführungsbeispiel ist als mittlerer optionaler Schrankteil noch der Kühl-Schrankteil 10 vorgesehen, in dem eine so genannte Wärmerückkühlanlage 26 angeordnet ist, die zur Klimatisierung des gesamten Analyseschranks 2 dient. Diese ist nach Art einer herkömmlichen Kälte- oder Klimaanlage ausgestaltet und umfasst einen Kältekreislauf. Die Wärmerückkühlanlage 26 - von der nur einige wenige Komponenten dargestellt sind - umfasst dementsprechend auch mehrere Wärmetauscher 28 zur Kühlung. Die aufgenommene Wärme wird über einen weiteren Wärmetauscher 30, welcher im Ausführungsbeispiel durch einen auf der Oberseite des Analyseschranks 2 angeordneter Außenwärmetauscher gebildet ist, an die Umgebung abgegeben. Wie aus der Fig. 2 zu entnehmen ist, ist jedem der weiteren Schrankteile 4,6,8 ein Wärmetauscher 28 zugeordnet. Im Ausführungsbeispiel ist vorgesehen, dass diese Wärmetauscher innerhalb des Kühl-Schrankteils 10 angeordnet sind. Alternativ besteht auch die Möglichkeit, die Wärmetauscher 28 über entsprechende Durchführungen in den einzelnen Schrankteilen 4,6,8 zu platzieren.

Wie in Fig. 2 durch die verdickte Umrandungslinie für den Elektro-Schrankteil 4 sowie auch weiterhin für den Analyse-Schrankteil 8 dargestellt ist, sind diese Schrankteile, insbesondere der Elektro-Schrankteil 4, als separate, abgetrennte und abgeschottete Schrankteile 4,6 ausgebildet. Hierdurch ist ein Kontakt mit den in der Raumluft außerhalb dieser Schrankteile 4,6 befindlichen gasförmigen oder flüssigen Stoffe vermieden. Unter Abschottung dieser Schrankteile 4,8 wird daher insbesondere eine zumindest weitgehend gasdichte Abdichtung verstanden, so dass innerhalb des Elektro-Schrankteils 4 als auch im Analyse-Schrankteil 8 ein von der Umgebung unbeeinflusstes Raumklima eingestellt ist. Durch diese Maßnahme ist gewährleistet, dass sämtlich elektronischen Komponenten, wie beispielsweise Einspeise- und Verteilerpunkte, die sich unter Spannung befinden, in einem abgeschotteten Raum angeordnet sind und insbesondere beispielsweise die EG-Richtlinie Niederspannung 2006/95/EG erfüllt sind.

Durch den speziellen Aufbau des Analyseschranks 2 mit dem abgeschotteten Elektro-Schrankteil 4 sowie vorzugsweise ergänzend auch mit dem abgeschotteten Analyse-Schrankteil 8 wird daher eine insgesamt in sich abgeschlossene Baueinheit bereitgestellt, die in sich sowohl die Gasaufbereitung als auch die Analyse des Messgases G aufnimmt und gleichzeitig so ausgelegt ist, dass sämtliche Normen und Richtlinien trotz der Kombination der unterschiedlichen Teilbereiche innerhalb eines gemeinsamen Analyseschranks 2 erfüllt ist.

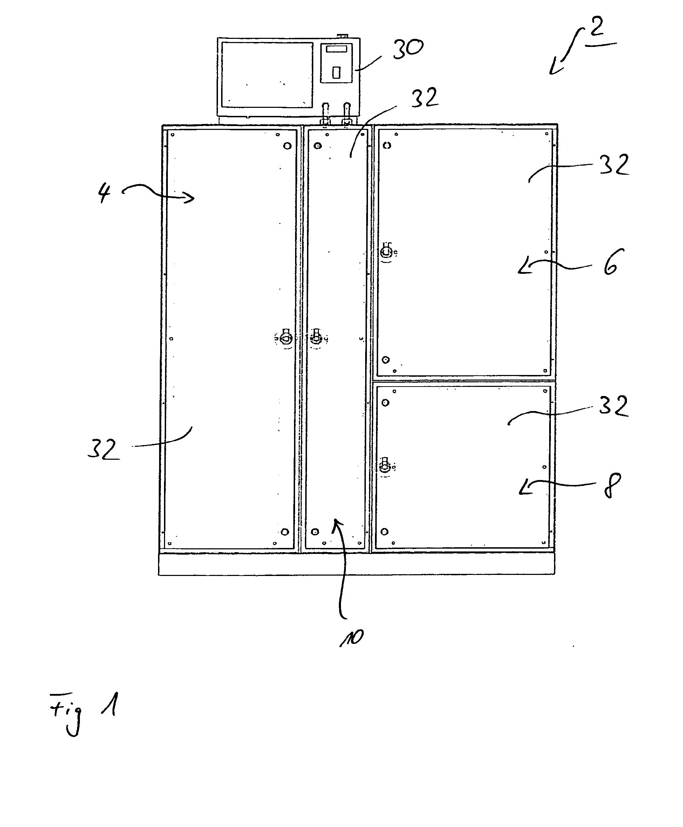

Im Hinblick auf den konstruktiven Aufbau weist der Analyseschrank 2 ein gemeinsames, hier nicht näher dargestelltes Traggerüst auf, welches üblicherweise durch miteinander verbundene Profilschienen gebildet ist. Durch dieses Traggerüst werden insbesondere die einzelnen Bereiche des Analyseschranks 2 untereinander abgetrennt. Weiterhin umfasst der Analyseschrank 2 ein gemeinsames Außengehäuse. Die einzelnen Schrankteile 4 bis 10 sind im Inneren durch Wände und Böden voneinander getrennt. Lediglich der Gas-Schrankteil 6 kann zum Kühl-Schrankteil 10 offen sein. Entscheidend ist, dass der Elektro-Schrankteil 4 und ergänzend vorzugsweise auch der Analyse-Schrankteil 8 durch (Schott-)Trennwände zu den benachbarten Schrankteilen sowie zur äußeren Umgebung hin möglichst gasdicht abgetrennt sind. Jedem Schrankteil 4,6,8,10 ist eine Tür 32 zugeordnet, die sich jeweils - wie aus Fig. 1 hervorgeht - über die gesamte Frontseite des jeweiligen Schrankteils 4 bis 10 erstreckt.

Insgesamt kennzeichnet sich der Analyseschrank 2 durch seine integrale Ausgestaltung mit der Gasaufbereitung sowie der Gasanalyse und dem getrennten Elektro-Bereich innerhalb eines gemeinsamen Schrankgehäuses bei gleichzeitiger kompakter Bauform aus. Durch die Abschottung insbesondere des Elektro-Schrankteils 4 und durch die Anordnung des Gas-Schrankteils 6 zur Gasaufbereitung ist die Einhaltung unterschiedlichster Normen, insbesondere der EG-Maschinenrichtlinie sichergestellt. Gleichzeitig ist eine Reduzierung des Gefahrenpotenzials bezüglich der Elektronik bei Kontakt mit anderen Medien gewährleistet. Insgesamt ist dadurch eine hohe Betriebssicherheit erreicht.

### Bezugszeichenliste

- 2: Analyseschrank
- 4: Elektro-Schrankteil
- 6: Gas-Schrankteil
- 8: Analyse-Schrankteil
- 10: Kühl-Schrankteil
- 12: Gasaufbereitungsvorrichtung
- 14: Analyseeinrichtung
- 16A: Gasdurchführung
- 16B: Gasdurchführung
- 18A: Trennwand
- 18B: Trennwand
- 20: Schaltkastenanordnung
- 22: Leitung
- 24: Elektrodurchführung
- 26: Wärmerückkühlanlage
- 28: Wärmetauscher
- 30: Wärmetauscher
- 32: Tür

- G: Messgas

## Patentansprüche

1. Analyseschrank (2) zur Analyse eines insbesondere aus einem großtechnischen Prozess stammenden Messgases (G), umfassend einen Elektro-Schrankteil (4), in dem eine elektrische Schaltkastenanordnung (20) vorgesehen ist, einen Gas-Schrankteil (6), in dem eine Gasaufbereitungsvorrichtung (12) angeordnet ist, sowie einen Analyse-Schrankteil (8), in dem eine Analyseeinrichtung (14) zur Messgasanalyse angeordnet ist, wobei der Elektro -Schrankteil (4) zu den weiteren Schrankteilen hin abgeschottet ist.

2. Analyseschrank (2) nach Anspruch 1,
bei dem ergänzend der Analyse-Schrankteil (8) abgeschottet ist.

3. Analyseschrank (2) nach Anspruch 1 oder 2,
bei dem zwischen dem Elektro-Schrankteil (4) und dem Gas- Schrankteil (6) eine dichte Trennwand (18B) mit einer abgedichteten Elektrodurchführung (24) für zumindest eine elektrische Leitung (22) vorgesehen ist.

4. Analyseschrank (2) nach einem der vorhergehenden Ansprüche,
bei dem zwischen dem Analyse-Schrankteil (8) und dem Gas-Schrankteil (6) eine dichte Trennwand (18B) mit einer abgedichteten Gasdurchführung (16A) für das aufbereitete Messgas (G) vorgesehen ist.

5. Analyseschrank (2) nach Anspruch 4,
bei dem der Analyse-Schrankteil (8) eine zweite abgedichtete Gasdurchführung (16B) für das Messgas (G) nach Durchlaufen der Analyseeinrichtung (14) aufweist, insbesondere zur Rückführung des Messgases (G) in den Gas-Schrankteil (6).

6. Analyseschrank (2) nach einem der vorhergehenden Ansprüche,
bei dem die Gasaufbereitungsvorrichtung (12) wahlweise oder in Kombinationen aufweist einen Gaswäscher, einen Partikelfilter, eine Einrichtung zur Be- oder Entfeuchtung des Messgases (G), einen Kondensatbehälter, Ventile sowie einen Kühler für das Messgas (G).

7. Analyseschrank (2) nach einem der vorhergehenden Ansprüche,
bei dem zusätzlich ein Kühl- Schrankteil (10) vorgesehen ist, in dem zumindest Teile einer Wärmerückkühlanlage (26) zur Temperierung des Analyseschranks (2) auf eine vorgegebene Betriebstemperatur angeordnet sind.

8. Analyseschrank (2) nach Anspruch 7,
bei dem der Kühl- Schrankteil (10) angeordnet ist zwischen dem Elektro-Schrankteil (4) einerseits und dem Gas- (6) sowie Analyse-Schrankteil(8) andererseits, die übereinander positioniert sind.

9. Analyseschrank (2) nach Anspruch 7 oder 8,
bei dem die Wärmerückkühlanlage (26) für mehrere der Schrankteile (4,6,8,10) einen jeweiligen Wärmetauscher (28) aufweist, der jeweils an einer Trennwand (16B) zum jeweiligen Schrankteil (4,6,8,10) anliegt.

10. Analyseschrank (2) nach einem der vorhergehenden Ansprüche,
bei dem die einzelnen Schrankteile(4,6,8,10) jeweils über Türen (32) zugänglich sind.

11. Verfahren zur Analyse eines Messgases (G), das aus einem großtechnischen Prozess stammt und insbesondere ein Abgas eines Verbrennungsprozesses ist, mit Hilfe eines Analyseschrankes (2) nach einem der vorhergehenden Ansprüche.
